# EUROPEAN PATENT SPECIFICATION

(11) **EP 3 291 784 B1**
(45) Date of publication and mention of the grant of the patent: **01.05.2019**
(21) Application number: 16724858.2
(22) Date of filing: 04.05.2016
(51) Int. Cl.: A61F 13/51, D04H 1/4391, D04H 1/544, D04H 3/018, D04H 3/14

(54) **NON-WOVEN FABRIC**
VLIESSTOFF
ÉTOFFE NON TISSÉE

(30) Priority: 06.05.2015 EP 15166615
(43) Date of publication of application: 14.03.2018
(73) Proprietor: Fitesa Germany GmbH, 31224 Peine (DE)
(72) Inventor: SIEBNER, Harald, 38122 Braunschweig (DE); HARTL, Helmut, 38124 Braunschweig (DE); NOVARINO, Elena, 31224 Peine (DE)
(74) Representative: van Wijk, Alexander Pieter
(86) International application number: PCT/EP2016/060012
(87) International publication number: WO 2016/177786

(56) References cited:
- EP-A1- 1 722 020
- EP-A1- 2 821 043
- WO-A1-2012/125701
- WO-A1-2012/130414
- CN-U- 201 785 556
- DE-A1- 3 634 146
- US-A- 3 508 390
- US-A- 5 208 106
- US-A1- 2004 092 192
- US-A1- 2008 032 579

## Description

### FIELD OF THE INVENTION

The present invention relates to a nonwoven fabric, an absorbent article comprising the non-woven fabric, the use of the nonwoven fabric in an absorbent article, in particular a hygiene absorbent article, and an absorbent article comprising the nonwoven fabric.

### BACKGROUND OF THE INVENTION

Nonwoven fabrics are widely applied in disposable absorbent articles for personal care or hygiene, such as diapers, feminine care articles, incontinence articles and children's training pants. Whilst the main focus of such hygienic absorbent articles is the ability to absorb and retain bodily fluids, the visibility of a bonding pattern has also become important since it increases the attractiveness of the absorbent article. Especially for consumers it increases the perception of the softness of the absorbent article, and increases the aesthetic perception of geometric patterns on the fabric's surface.

In EP 2821043 A1, nonwoven fabrics are described which are formed from fibers that preferably have a round cross-section. Such nonwoven fabrics have the drawback, however, that the visibility of the bonding patterns leaves much room for improvement, particularly at lower basis weights.

Object of the present invention is to provide a nonwoven fabric from which components for absorbent articles, in particular hygiene absorbent articles, can be made, which display an improved visibility of the bonding pattern and are therefore are highly attractive to consumers.

### SUMMARY OF THE INVENTION

It has now been found that this can be established when use is made of nonwoven fabrics comprising a nonwoven web which is formed from a particular type of fibers and which has a low basis weight.

Accordingly, the present invention relates to a nonwoven fabric as defined in claim 1.

A major advantage of the present invention resides in the fact that the present nonwoven fabric has a bonded pattern with an improved visibility when compared with nonwoven fabrics that are formed from round fibers which are conventionally used and which have a similar basis weight.

### DETAILED DESCRIPTION OF THE INVENTION

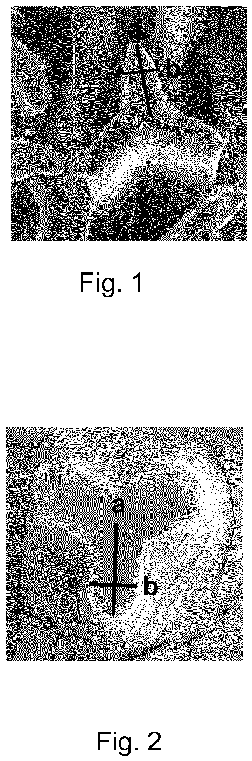

In accordance with the present invention the nonwoven fabric comprises a nonwoven web which is formed from a plurality of trilobal fibers, wherein the lobes of the trilobal fibers each have a ratio of length to width (L/W) in the range of from 2.2-4. Preferably, the lobes of the trilobal fibers each have a ratio of length to width in the range of from 2.5-4. In the context of the present invention the length of a lobe means the distance between the end point of a lobe and the center of the core of the trilobal fiber (see also lines (a) in Figures 1 and 2). The width is defined as the average distance between the two opposite sides of an individual lobe (see also lines (b) in Figures 1 and 2).

Suitably, in accordance with the present invention the trilobal fibers are made from thermoplastic polymers. The nonwoven webs particularly suitable are made from fibers of thermoplastic polymers such as polyolefins, polyesters, or polyamides, and combinations thereof, but may also comprise natural fibers such as wood, cotton, or rayon in combination with thermoplastic fibers. The nonwoven web may also be a composite made up of a mixture of two or more different fibers or a mixture of fibers and particles.

The fibers are suitably joined by bonding to form a coherent web structure. Suitable bonding techniques include, but are not limited to, chemical bonding and thermal bonding, for example thermal calendering or bonding by a hot gas stream.

Particularly preferred are polyolefin fibers. A wide range of suitable polyolefins can be used in the present invention. Suitable examples include polyethylene, polypropylene, copolymers of ethylene with butene, hexene, or octene, copolymers of polypropylene and ethylene. The polyolefins may comprise a homopolymer or a copolymer such as propylene-α-olefins copolymers. In particularly, the latter copolymers can attractively be used in the present invention. Preferred are polyolefin materials that comprise a propylene-α-olefin copolymer and a propylene homopolymer.

The melt flow rate (MFR) of the polyolefin material is suitably less than 90 dg/min. The MFR is determined using ASTM test method D1238, 2.16 kg. Preferably, the MFR of the polyolefin material is in the range of from 15-50 dg/min, more preferably in the range of from 15-35 dg/min.

Preferably, the fibers are formed from polyethylene or polypropylene homopolymers, co-polymers thereof, blends of polyethylene and polypropylene, a polyester, co-polymers of polyesters and/or blends of polyesters.

Suitably use is made of a propylene-based or ethylene-based homopolymer or a copolymer. In the case of propylene-based polymers the polymers may comprise comonomer-derived units selected from ethylene and C4-C10 α-olefins. In the case of ethylene-based polymers the polymers may comprise comonomer-derived units selected from C3-C10 α-olefins. Suitable examples of polyolefin materials include propylene homopolymers, ethylene homopolymers, propylene copolymers and ethylene copolymers such as linear low density polyethylene (LLDPE), high density polyethylene (HDPE), and low density polyethylene (LDPE).

Suitable polyesters can be aliphatic polyesters such as, e.g. polylactic acid, or aromatic polyesters such as polyethylene terephthalate (PET) and poly(trimethylene terephthalate) (PTT).

Besides additives already contained in the employed polymers, addition of further additives is possible to provide additional properties to the fibers. Suitable further additives include thermal stabilizers, light stabilizers, slip additives, waxes, and additives to make the fabrics either hydrophilic or hydrophobic. The addition of filler materials can sometimes also be of advantage. Suitable filler materials include organic and inorganic filler materials. Suitable examples of inorganic filler materials include minerals such as calcium carbonate, metals such as aluminum and stainless steel. Suitable examples of organic filler materials include sugar-based polymers. The trilobal fibers to be used in accordance with the present invention can suitable be single component or multicomponent fibers such as bicomponent fibers. In case use is made of bicomponent fibers the lobes will be made of one type of polymer and the core to which the lobes are attached will be made of another type of polymer. Most preferred are core-sheath bicomponent fibers comprising polyethylene and polypropylene, but any other combination of other suitable polymers is possible as well, for example combinations of polyesters with polyolefins. The bicomponent fibers may contain different types of polypropylene. More preferably, the bicomponent fiber has a core of a polypropylene which has a higher melting point and lobes of a polypropylene which has a lower melting point. In another embodiment, the bicomponent fiber comprises two polypropylenes that differ in melt temperature or melt flow.

The fibers can be made according to spinning technologies known in the art. Most conveniently employed are spunbond processes, from which the nonwoven fabrics can directly be formed.

Spunbond fibers are generally produced by extruding a molten polymer through a large spinneret having several thousand holes per linear meter or from banks of smaller spinnerets, for example, containing as few as 40 holes. After exiting the spinneret, the molten fibers are quenched by a cross-flow air quench system, then pulled away from the spinneret and attenuated by high speed air. Lay-down of the filaments to create a nonwoven layer occurs on a permeable transport belt. Spunbond fibers are generally continuous and range in fiber diameter between ca. 10-100 µm.

The non-woven fabric in accordance with the invention can additionally be treated to add specific properties. Most common are topical treatments to make the fabric either hydrophilic or to make it hydrophobic. Most common is the treatment of the fabric with either hydrophilic surfactants or with a fluorocarbon or a silicon material. In the context of the present invention a surface of a non-woven fabric or non-woven web is "hydrophilic" when the contact angle of water disposed on that surface is less than about 90 and a surface is "hydrophobic" when the contact angle of water disposed on that surface is greater than or equal to 90.

Preferably, the nonwoven fabrics in accordance with the invention are hydrophobic non-woven fabrics.

The nonwoven fabric according to the invention can consist of only one type of fibers or fiber layers, e.g. a spunbond layer, but it suitably can comprise additional fiber layers which may be different. Suitable multi-layer fabrics, for example, may include one or more spunbond layers (S) and one or more meltblown layers (M), such as SMS, SMMS, SSMMS, etc. adhered to form a nonwoven fabric according to the present invention. Usually, these multilayer fabrics are made in one step on a single line with multiple beams, which generally encompass a combination of spunbond and meltblown beams. In some cases it might be advantageous or technically necessary to make a multiple layer according to the invention in two or more separate steps.

Combination of spunbond layers with natural fibers is possible as well. Preferably, the additional nonwoven webs to be used in accordance with the present invention are made of meltblown fibers.

A major advantage of the present invention is that nonwoven fabrics are provided with bonded patterns having an improved visibility when compared with round fibers that are conventionally used. This improvement is established by using particular trilobal fibers in combination with a low basis weight of the nonwoven web. The nonwoven web to be used in accordance with the present invention has a basis weight in the range of from 8-16 gsm (g/m²). The basis weight can be determined in accordance with DIN EN 29073-1.

Suitably, the nonwoven web according to the present invention has a tensile strength according WSP 110.4 in MD (Machine Direction) in the range of from 1-4 N per gram basis weight, preferably in the range of from 1.2-3.5 N per gram basis weight, and more preferably in the range of from 1.3-3.0 N per gram basis weight. Non-woven webs with such tensile strengths provide non-woven articles with a high tensile strength.

This WSP test method is an internationally acknowledged test method in the non-woven's industry, as the skilled person will understand.

The present nonwoven fabrics are suitably made from fibers that have a weight in the range of from 1-6 dtex, preferably in the range of from 1.5-5 dtex, and more preferably in the range of from 1.8-4 dtex.

Suitably, the nonwoven webs to be used in accordance with the present invention comprise at most 25 wt% of meltblown fibers, based on total weight of the non-woven web. Preferably, the non-woven webs comprises at most 20 wt% of meltblown fibers, based on the total weight of the non-woven web.

Suitably, the present nonwoven webs contain spunbond fibers only, not mixtures of spunbond fibers and another type of fibers.

The skilled person will understand that the major part of the nonwoven fabric will consist of the fibers. Suitably, at least 90 wt% of the nonwoven fabric is made of the fibers, preferably at least 95 wt% of the nonwoven fabric is made of the fibers, and even more preferably at least 98 wt% of the nonwoven fabric is made of the fibers.

The nonwoven fabrics in accordance with the present invention comprise a nonwoven web which has a side which is provided with a pattern of bonded areas which defines one or more non-bonded areas, and wherein the surface of the bonded areas is in the range of 10-32 % of the total surface of the side and the surface of the non-bonded area(s) is in the range of from 68-90 % of the total surface of the side.

As a further advantage of the present invention, the trilobal fibers to be used according to the present invention allow a more open bonded pattern (lower bond area) compared with round fibers, which require denser bonded patterns to achieve the same degree of several physical properties, including stiffness. Such a more open bonded pattern beneficially supports the optical impression of higher softness, which is caused by better visibility of the bond pattern, by a higher fluffiness of the fabric as a direct result of the more open bonded area.

The high surface of the one or more non-bonded areas to be used according to the present invention provides an attractively high softness. Moreover, the large unbonded area(s) allow for the fiber to bulk up and increase the bulkiness of the fabric. This is perceived as an even higher softness from both visual and the tactile perspective. The surface of the non-bonded area(s) is in the range of from 68-90 of the total surface area of the side. Preferably, the surface of the non-bonded area(s) is in the range of from 75-85% of the total surface area of the side.

The surface of the bonded areas is preferably in the range of 10-32%, more preferably in the range of from 15-25 % of the total surface area of the side.

The nonwoven web to be used in accordance with the present invention is provided with a pattern of bonded areas which defines one or more non-bonded areas. Suitably, in accordance with the present invention the nonwoven web has a side which is provided with a first pattern of individualized bonded areas defines a second pattern of non-bonded areas..

The present invention therefore also relates to a nonwoven fabric as defined in claim 1.

The individualized bonded areas can suitably be uniformly arranged over the total surface of the side of the nonwoven web. Such patterns are used for bonding since many years and are known to someone skilled in the art.

The individualized bonded areas suitably have a symmetrical shape.

According to the invention, the individualized bonded areas have rod shapes in the cross direction (CD) of the nonwoven web.

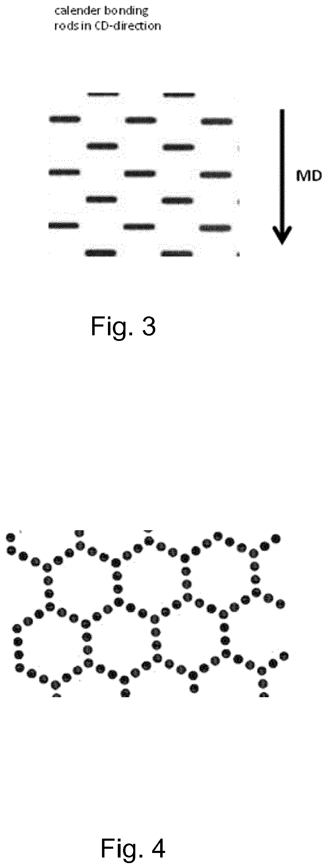

Figure 3 shows a nonwoven web not according to the invention where the pattern of bonded areas defines one large non-bonded area. This is for instance the case when the bonded areas all have the same shape and size such (e.g. rods in the cross direction of the nonwoven web) and are all evenly distributed over the side of the nonwoven web (see Figure 3). According to the invention, the pattern of bonded areas defines a pattern of a number of non-bonded areas, whereby the non-bonded areas may have various shapes. Suitable shapes of the non-bonded areas include squares, circles, and hexagons. Preferably, the non-bonded areas have a hexagonal shape (see Figure 4). The pattern of bonded areas can consist of individualized bonded areas all having the same shape or alternatively the individualized bonded areas may have different shapes. For instance, in the latter case a combination of circles and rods can be used. In yet another embodiment two or more patterns of individualized bonded areas can be used that define one or more patterns of non-bonded areas.

Preferably, the side of the non-woven fabric consists only of a first pattern of bonded areas which defines a second pattern of non-bonded areas, meaning that no further pattern of bonded or non-bonded areas is provided on the side of the non-woven fabric.

Suitably, the individualized bonded areas have a surface in the range of from 0.30-5.00 mm², preferably in the range of from 0.40-3.50 mm², and more preferably in the range of from 0.50-2.0 mm². The discrete non-bonded areas suitably have a depth in the range of from 0.4-1.5 mm, preferably in the range of from 0.4-0.9 mm, more preferably in the range of from 0.4-0.8 mm, and most preferably in the range of from 0.5-0.7 mm.

The present invention also relates to an absorbent article comprising a nonwoven fabric according to the present invention. Suitably, the absorbent article according to the present invention is a disposable hygiene absorbent article selected from the group consisting of incontinence articles, diapers, wipes and fem-care articles. Suitable disposable hygiene absorbent articles according to the present invention include those selected from the group consisting of baby diapers, pull-ups, training pants, adult incontinence briefs and diapers.

The nonwoven fabric according to the present invention can suitably be part of a top sheet and/or a back sheet.

The present invention further relates to the use of the nonwoven fabric according to the present invention in a reinforcement layer of an absorbent article.

The present invention also relates to the use of trilobal fibers in a nonwoven fabric which comprises a pattern of bonded areas for increasing the visibility of the pattern of bonded area, wherein the lobes of the trilobal fibers each have a ratio of length to width in the range of from 2.2-4.

The present invention also relates to the use of trilobal fibers to increase the visibility of a pattern of bonded areas which is present on a side of a nonwoven web, wherein the lobes of the trilobal fibers each have a ratio of length to width in the range of from 2.2-4.

The present invention also relates to the use of trilobal fibers in a nonwoven as defined in claim 1.

In Figures 1 and 2, pictures are shown of trilobal fibers. In Figure 1, a trilobal fiber according to the invention is shown with a ratio of length to width of 1:3, whereas in Figure 2 a trilobal fiber is shown with a ratio of length to width of 1:2 (not according to the invention).

### Examples

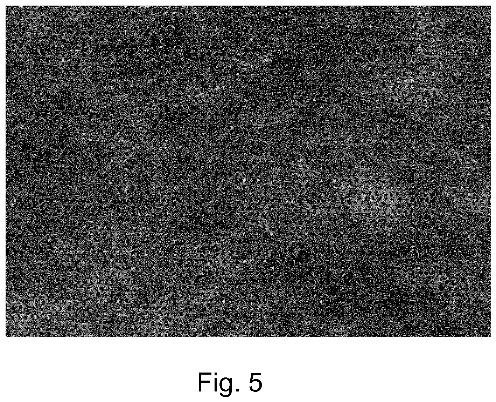

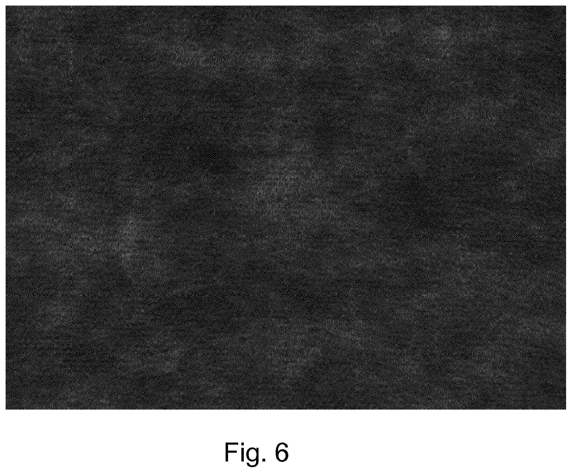

In these Examples a comparison is made between nonwoven fabrics made from round fibers and nonwoven fabrics made from trilobal fibers. The visibility of the bonded patterns of the respective nonwoven fabrics is shown in Figures 5 and 6.

### Example 1

In this Example, a nonwoven web is prepared by a nonwoven fabric made from trilobal fibers. The bonding pattern consisted of bonded areas having an elliptic shape. The surface of the bonded areas was 18 %, and the surface of the non-bonded area was 82%, based on the total surface of one of the sides of the nonwoven fabrics. Further, the basis weight was 12 gsm and the fiber titer was 2.4 dtex. The thickness of the trilobal fibers is defined as the distance between the end points of two respective lobes, and was 19 µm. In Figure 5, a picture is shown of the nonwoven web.

### Example 2 (Comparative Example)

In this Example, a nonwoven web is prepared by a nonwoven fabric made from round fibers. In this case the bonding pattern also consisted of bonded areas having an elliptic shape. The surface of the bonded areas was 18 % and the surface of the non-bonded area was 82%, based on the total surface of one of the sides of the nonwoven fabrics. Further, the basis weight was 12 gsm and the fiber titer was 2.0 dtex. The thickness of the round fibers and was 16.7 µm. In Figure 6, a picture is shown of the nonwoven web.

From Figures 5 and 6 it will be clear that the bonding pattern of the nonwoven web made from trilobal fibers is much more visible when compared with the nonwoven web which was prepared with round fibers.

## Claims

1. A nonwoven fabric comprising a nonwoven web which is formed from a plurality of trilobal fibers, wherein the lobes of the trilobal fibers each have a ratio of length to width in the range of from 2.2-4, the nonwoven web has a basis weight in the range of from 8-16 gsm, the nonwoven web has a side which is provided with a first pattern of individualized bonded areas which defines a second pattern of non-bonded areas, wherein the individualized bonded areas have a rod shape in the cross direction (CD) of the nonwoven web, and wherein the surface of the bonded areas is in the range of 10-32 % of the total surface of the side and the surface of the non-bonded area(s) is in the range of from 68-90 % of the total surface of the side.

2. The nonwoven fabric according to claim 1, wherein the lobes of the trilobal fibers each have a ratio of length to width in the range of from 2.5-4.

3. The nonwoven according to claim 1 or 2, wherein the trilobal fibers are made from thermoplastic polymers.

4. The nonwoven according to claim 3, wherein the thermoplastic polymers comprise polypropylene and/or polyethylene.

5. The nonwoven fabric according to any one of claims 1-4, wherein the trilobal fibers have a weight in the range of from 1.5-5 dtex.

6. Use of the nonwoven fabric according to any one of claims 1-5 in an absorbent article.

7. Use according to claim 6, wherein the absorbent article is a hygiene absorbent article selected from the group consisting of baby diapers, pull-ups, training pants, hygiene closure systems, adult incontinence briefs and diapers.

8. Use according to claim 7, wherein the nonwoven fabric forms at least part of the back sheet and/or top sheet of the hygiene absorbent article.

9. Use of trilobal fibers as defined in any one of claims 1-5 for increasing the visibility of a pattern of bonded areas which is present on a side of a nonwoven web.

10. An absorbent article comprising a nonwoven fabric according to any one of claims 1-5.

11. An absorbent article according to claim 10, wherein the absorbent article is a hygiene absorbent article selected from the group consisting of baby diapers, pull-ups, training pants, hygiene closure systems, adult incontinence briefs and diapers.

## Patentansprüche

1. Vliesstoff, der eine Vliesbahn umfasst, die aus mehreren trilobalen Fasern gebildet ist, wobei die Loben der trilobalen Fasern jeweils ein Verhältnis von Länge zu Breite im Bereich von 2,2-4 aufweisen, wobei die Vliesbahn ein Flächengewicht im Bereich von 8-16 g/m² aufweist, wobei die Vliesbahn eine Seite aufweist, die mit einem ersten Muster von individualisierten gebundenen Bereichen versehen ist, das ein zweites Muster von nicht-gebundenen Bereichen definiert, wobei die individualisierten gebundenen Bereiche eine Stabform in der Querrichtung (CD) der Vliesbahn aufweisen und wobei die Oberfläche der gebundenen Bereiche im Bereich von 10-32 % der Gesamtoberfläche der Seite liegt und die Oberfläche des bzw. der nicht-gebundenen Bereiche im Bereich von 68-90 % der Gesamtoberfläche der Seite liegt.

2. Vliesstoff nach Anspruch 1, wobei die Loben der trilobalen Fasern jeweils ein Verhältnis von Länge zu Breite im Bereich von 2,5-4 aufweisen.

3. Vliesstoff nach Anspruch 1 oder 2, wobei die trilobalen Fasern aus thermoplastischen Polymeren hergestellt sind.

4. Vliesstoff nach Anspruch 3, wobei die thermoplastischen Polymere Polypropylen und/oder Polyethylen umfassen.

5. Vliesstoff nach einem der Ansprüche 1-4, wobei die trilobalen Fasern ein Gewicht im Bereich von 1,5-5 dtex aufweisen.

6. Verwendung des Vliesstoffs nach einem der Ansprüche 1-5 in einem absorbierenden Artikel.

7. Verwendung nach Anspruch 6, wobei der absorbierende Artikel ein absorbierender Hygieneartikel ist, der aus der Gruppe bestehend aus Babywindeln, Pull-Ups, Trainerhöschen, Hygieneverschlusssystemen, Inkontinenzslips für Erwachsene und Windeln ausgewählt ist.

8. Verwendung nach Anspruch 7, wobei der Vliesstoff mindestens einen Teil des Rückenblatts und/oder des Deckblatts des absorbierenden Hygieneartikels bildet.

9. Verwendung von trilobalen Fasern wie in einem der Ansprüche 1-5 definiert zur Erhöhung der Sichtbarkeit eines Musters von gebundenen Bereichen, die auf einer Seite einer Vliesbahn vorliegen.

10. Absorbierender Artikel, der einen Vliesstoff nach einem der Ansprüche 1-5 umfasst.

11. Absorbierender Artikel nach Anspruch 10, wobei der absorbierende Artikel ein absorbierender Hygieneartikel ist, der aus der Gruppe bestehend aus Babywindeln, Pull-Ups, Trainerhöschen, Hygieneverschlusssystemen, Inkontinenzslips für Erwachsene und Windeln ausgewählt ist.

## Revendications

1. Étoffe non tissée comprenant un voile non tissé qui est formé à partir d'une pluralité de fibres trilobées, les lobes des fibres trilobées ayant chacun un rapport longueur/largeur dans la plage allant de 2,2 à 4, le voile non tissé ayant une masse surfacique dans la plage allant de 8 à 16 g/m², le voile non tissé ayant un côté qui comporte un premier motif de zones liées individualisées qui définit un second motif de zones non liées, les zones liées individualisées ayant une forme de tige dans le sens travers (CD) du voile non tissé, et la surface des zones liées étant dans la plage allant de 10 à 32 % de la surface totale du côté et la surface de la ou des zones non liées étant dans la plage allant de 68 à 90 % de la surface totale du côté.

2. Étoffe non tissée selon la revendication 1, dans laquelle les lobes des fibres trilobées ont chacun un rapport longueur/largeur dans la plage allant de 2,5 à 4.

3. Étoffe non tissée selon la revendication 1 ou 2, dans laquelle les fibres trilobées sont réalisées à partir de polymères thermoplastiques.

4. Étoffe non tissée selon la revendication 3, dans laquelle les polymères thermoplastiques comprennent du polypropylène et/ou du polyéthylène.

5. Étoffe non tissée selon l'une quelconque des revendications 1 à 4, dans laquelle les fibres trilobées ont un poids dans la plage allant de 1,5 à 5 dtex.

6. Utilisation de l'étoffe non tissée selon l'une quelconque des revendications 1 à 5 dans un article absorbant.

7. Utilisation selon la revendication 6, dans laquelle l'article absorbant est un article absorbant hygiénique choisi parmi le groupe constitué de couches pour bébés, de couches-culottes, de culottes de propreté, de systèmes de fermetures hygiéniques, de culottes et de couches d'incontinence pour adultes.

8. Utilisation selon la revendication 7, dans laquelle l'étoffe non tissée forme au moins une partie de la feuille arrière et/ou de la feuille supérieure de l'article absorbant hygiénique.

9. Utilisation de fibres trilobées définies selon l'une quelconque des revendications 1 à 5 pour augmenter la visibilité d'un motif de zones liées qui est présent sur un côté d'un voile non tissé.

10. Article absorbant comprenant une étoffe non tissée selon l'une quelconque des revendications 1 à 5.

11. Article absorbant selon la revendication 10, l'article absorbant étant un article absorbant hygiénique choisi parmi le groupe constitué de couches pour bébés, de couches-culottes, de culottes de propreté, de systèmes de fermetures hygiéniques, de culottes et de couches d'incontinence pour adultes.
